# EUROPEAN PATENT APPLICATION

(11) **EP 4 697 345 A1**
(43) Date of publication of application: **18.02.2026**
(21) Application number: 24195076.5
(22) Date of filing: 17.08.2024
(51) Int. Cl.: G16H 20/60, G16H 40/67

(54) **DYNAMIC FOOD RATING DETERMINING SYSTEM USING USER PROGRAMMED FUNCTIONS**

(71) Applicant: Bajer, Maciej, 01-864 Warsaw Mazowieckie (PL)
(72) Inventor: Bajer, Maciej, 01-864 Warsaw Mazowieckie (PL)

(57) **Abstract**

The invention relates to a computer system, which calculates personalized rating for a food product, based on multi-domain parameters and user programmed functions. System comprises user device with client application, a server with server application, and a connection between user device and a server. System uses user programmed diet configurations and user programmed diet events to process food product analysis. Diet configuration is a set of conditions and rules which is used for every day usage. Diet event is a set of conditions and rules which is used for specific, activated moment using multi-domain activation factor. System assists user in making decisions about consuming food and managing food product consumptions. This invention relates generally to information engineering and dietetics.

## Description

### FIELD OF INVENTION

The invention relates to a computer system, in a computer network, which calculates personalized rating for a food product, based on multi-domain parameters and user programmed functions. System assists user in making decisions about consuming food and managing food product consumptions. This invention relates generally to information engineering and dietetics.

### BACKGROUND OF INVENTION

Food product rating is an essential way to inform about the quality of the edible product. It may have big impact on health or life, depending on the health condition of a person.

The earliest food ratings were effective for their simplicity, but as technology progressed they remained unchanged for modern solutions. Today it is common to find static food ratings prepared by national or international health organizations. Such ratings often require recalculating rating for each individual person considering person attributes such as gender, weight, age or others. The person must undergo the cumbersome steps to get a rating which is valid for their type of predefined, average human model.

Furthermore predefined ratings are invalid for non-average people. Such person may have completely different dietary needs or health issues. This person is forced to manually calculate the food rating on their own, all along.

There is need to use personalized set of rules to generate personalized food rating. Moreover there is need to use temporary set of rules depending of personalized factors (e.g. different rules for specific time interval or specific location).

For example US8647121B1, US2009/016446 and US8504385B2 relate to evaluating food rating with provided user profile data. Each of them may use nutritional values and included ingredients in a food product. However they lack analyzing other objective food data (e.g. food mass, energy, volume, dimensions, allergens and others), subjective food data (e.g. taste, odor, food hardness and others) and non-food data (e.g. time of analysis, location of analysis, history of user's consumptions, user state of mind). They evaluate food in a predefined way, which cannot be changed or adapted to make food ratings personalized.

For example US11244752B2 and US12046350B2 relate to filtering and recommending foods with provided user profile data, using machine-learning model. As well as previous inventions they also lack analyzing other objective food data, subjective food data and non-food data. Moreover they also evaluate food in a predefined way, based on a trained machine-learning model, which cannot be changed to personalize food ratings for each user individually.

There is no solution to get fully personalized food rating. Food ratings are generated only on a predefined way. For example eating a lot of carbohydrates is generally bad for health, but for a specific person (e.g. professional athlete) it could be necessary to stay in a good health.

There is no solution that process other food data than nutrients or ingredients. It causes that food product ratings are archaic and shallow. They lack a lot of details, depth and personalization which is defined by a unique life of every person.

### SUMMARY

The invention is defined by the appended independent claims. Additional features, uses and advantages of the concepts disclosed herein are set forth in the description which follows, and in part will be obvious from the description, or may be learned by practice of the described technologies.

The present disclosure describes system for dynamically calculating food product rating. System comprises a user device, a server, and a network connection between the user device and the server enabling data transfer between them. The user device runs a client application. The server runs a server application.

The present disclosure includes 2 analysis processing methods based on the device type: the user device and a server.

The present disclosure describes 2 analysis types depending on object with user programmed functions: diet configuration or diet event. Both of them can be created and updated by the user. Both of them comprises sets of conditions and rules for analysis processing. Additionally diet event contains activation factor or factors to make this diet event activated and presented to user.

The current solutions for calculating food product rating do not consider the impact of the amount of the food product on human health, the impact of the time (moment) of consumption and possible changes in the physical, chemical or biological parameters of the food product:
- The advantage of the invention is that it allows to determine the personalized quality rating of a food product for a person in an automated manner.
- The advantage of the invention is that it allows to process many different objective and subjective food product attributes in the food product analysis.
- The advantage of the invention is that it allows to process non-food data in the food product analysis.
- The advantage of the invention is that it allows to include specific and personalized features in the food product analysis according to user programmed functions.
- The advantage of the invention is that it allows to obtain ratings of many different products in parallel at the same time.
- The advantage of the invention is that it allows to take the change of physical, chemical and biological parameters of the analyzed food product into account in the food product analysis.

This allows for faster, easier and more precise monitoring of the food products consumption that is crucial to keep good shape and health for every person.

In addition, the system allows you to monitor product consumption using diet events:
- The advantage of the invention is that it allows to determine the quality of food product during temporary event, which has never been developed before. When the quality of food product is known during specific diet event, the user can change future decisions regarding their diet (e.g. increase the consumption of a specific type of product later this day or completely eliminate the consumption of a specific type of product for next week).
- The advantage of the invention is that it allows to program temporary events for complex diets.
- The advantage of the invention is that it allows to integrate user's diet with other aspects of life using activation factors in diet events.

### DETAILED DESCRIPTION

The invention comprises a user device, a server, and a network connection between the user device and the server enabling data transfer between them. The user device is a computer-type device. The user device comprises a processor, input-output devices, operating memory, and persistent memory. The server comprises a processor, input-output devices, operating memory, and persistent memory. The user device runs a client application. The server runs a server application. This invention relies on processing food product or food products data and generating the result of analysis.

The user can obtain the result of food product analysis in 2 possible ways:
a) client-based analysis processing - the user device downloads necessary data from server for food product analysis, process food product analysis and presents the result of the food product analysis.
b) server-based analysis processing - the user device triggers the food product analysis process on server, downloads the result of the food product analysis and presents it.

Moreover, the user can analyze one or many products simultaneously towards 2 defined data types:
a) diet configuration - a description of diet independent of any activation factors.
b) diet event - a description of diet determined by activation factor (primarily a time trigger, but it may be another trigger such as the user's geographic location, the user's current health status, or the user's current financial status).

Both the analysis towards diet configuration and the analysis towards diet event is possible to concern one single product or many different products at the same time.

The user device runs a client application. The client application is responsible for communicating with the server in order to analyze food product. The client application has 2 operating modes for the following system operating modes:
a) client-based analysis result generation - mode of data downloading and local food product analysis. The application downloads the necessary data from the server needed to analyze the food product and processes the product analysis locally on the user device.
b) server-based analysis result generation - mode of downloading the result of analysis. The application downloads the food product analysis result from the server, which is generated on the server.

The server application of the system is running on the server. The server application is responsible for communicating with the client application (user). The server application has 2 operating modes for the following system operating modes:
a) client-based analysis result generation - mode of sending data necessary to perform food product analysis calculations. The server application sends the necessary data for food product analysis to the client application. The analysis is processed locally on the user device.
b) server-based analysis result generation - mode of locally analyzing the food product and sending the analysis result. The application processes the analysis of the food product on the server and sends the result of the analysis (without providing the data necessary to perform the calculations).

Both the client application and the server application is able to store locally in the device:
- file of the diet configuration
- file of the diet event
- file of the food product description

This symmetrical design allows to use one of the 2 mentioned above system operation modes depending on the purpose of use and hardware capabilities.

The server has a database of food products data. Each food product has an identifier that allows to quickly find a file of food product description. Food products data can be stored outside server database (for example as a QR code printed on paper sheet in restaurant). Each food product is described by objective parameters (such as mass of the portion of food or nutritional content) and subjective parameters (such as taste characteristics or smell intensity).

Diet configuration is a file that defines the user's dietary parameters. Diet configuration consists of a list of multiple sets of sentences that comprehensively define the user's diet. Diet configuration has 2 types of sentences:
a) regulation - a set of sentences that generates the component result of the analysis.
b) toleration - a set of sentences that decides which regulations and tolerances are to be active and which are to be included in the final food product analysis result.

The regulation consists of 2 subsets:
a) logical conditions - it is a set of logical sentences connected by logical connectors, where the number of logical sentences can be 0 (the empty set can occur).
b) logical rules - it is a set of logical sentences connected by logical connectors, where the number of logical sentences must be greater than 0 (the empty set cannot occur).

In addition, the regulation has defined intermediate values for each possible outcome:
a) success - the conditions and the rules are fulfilled.
b) fail - conditions are fulfilled and the rules are not fulfilled.
c) undetermined - the product does not have the data necessary to check the fulfillment of conditions or rules.

The defined intermediate values can be edited and set by the user to any other values. The intermediate values are used to get final result of the food product analysis. By default, the following intermediate values can be assumed:
a) success = +1
b) fail = -1
c) undetermined = 0

The toleration consists of 2 subsets:
a) logical conditions - it is a set of logical sentences connected by logical connectors, where the number of logical sentences must be greater than 0 (the empty set cannot occur).
b) references to regulations and tolerances - it is a list of regulation and tolerance identifiers that are to be canceled and excluded from the final food product analysis result if the current tolerance conditions are fulfilled.

A logical sentence (both in the set of logical conditions and logical rules) is the smallest unit of calculation in the analysis process. The logical sentence consists of 3 elements:
a) subject - a feature that is the subject of analysis in a logical sentence.
b) operation - mathematical operation between an subject and a value.
c) value - a defined simple value or a set of simple values.

Examples of food product features that can be a subject in a logical sentence are:
a) mass of food product portion
b) mass of carbohydrates in food product portion
c) average energy of a food product portion per 100g of food

Examples of features not related to food product that can be a subject in a logical sentence are:
a) current day of the week
b) current clock time
c) geographic location of the user
d) financial status of the user
e) user's health condition
f) number of medications taken today
g) user's state of well-being
h) amount of energy consumed in the last 24 hours

Examples of value types that can be a value in a logical sentence are:
a) a simple value: number, string, boolean value
b) a set of simple values: set of numbers, set of strings, set of boolean values

Examples of logical connectors between sentences A and B are:
a) A ∧ B - logical operation AND
b) A ∨ B - logical operation OR

The user can assign a selected diet configuration to their account. The user can view their diet configuration in the client application at any time and trigger a food product analysis for the selected diet configuration.

Food product analysis towards diet configuration can be performed depending on the system operation mode: on the user device or on the server. The analysis process towards diet configuration consists of the 3 phases:
a) downloading necessary data for analysis
b) first iteration of data processing
c) second iteration of data processing

The first phase is downloading necessary data for food product analysis. Regardless of where the analysis is processed (on the client device or on the server), the necessary data must first be downloaded. The minimum set of data needed is:
a) diet configuration
b) food product description

Depending on your system settings and configuration settings, it may be possible or necessary to download additional data (for example: the user's history of food consumption over past few days, the user's health status, the user's geographic location, and more).

The next phase is the first iteration of data processing. For each regulation and tolerance defined in the diet configuration, intermediate results are calculated. For each regulation, a conditions fulfillment result and a rules fulfillment result are determined. For each tolerance, a conditions fulfillment result are determined. In the case of an empty set of conditions, the entire regulation is always activated and calculations of the fulfillment of the rules are performed.

Determining the fulfillment of a single condition or rule comes down to checking the truth of a logical sentence. Example of condition or rule is:
a) food portion mass < 100 g
b) today = first week of the month
c) food color list contains "green"
d) amount of carbohydrates in 100 g of food > 15%

Each such logical sentence is calculated and the result is determined for 3 states:
a) success - sentence is true
b) fail - sentence is false
c) undetermined - the sentence is impossible to determine (e.g. the food product does not have the necessary data for analysis)

In the regulation, the results of conditions and rules are then calculated as a whole using logical connectors. For example for sentence A (which has state "success") and sentence B (which has state "undetermined"):
a) A ∧ B - result is false
b) A ∨ B - result is true
c) B ∧ B - result is false
d) B ∨ B - result is false

This means that for a set of sentences to be true, it must contain at least 1 true sentence. In this way, for each set of conditions and each set of rules, the result of fulfilling the set of sentences is calculated. At the end of this phase, each regulation and each tolerance has its own intermediate result of conditions and rules fulfillment.

The last phase of the analysis process is to determine the final result of the food product analysis. Iteration goes through all tolerances starting from the last one and ending with the first one. For each tolerance, the result of conditions fulfillment is checked. If the tolerance conditions are fulfilled, then this tolerance is activated. It means that the regulations and tolerances defined in this tolerance are being canceled from food product analysis. If the next tolerance on the list is "cancelled" it is not taken into account in the analysis and this tolerance is omitted. This means that the further on the list a tolerance is defined, the higher priority it has (because it can cancel the tolerances that are before it, unless it is canceled itself). After iterating through all tolerances and marking regulations and tolerances as canceled, the final food product analysis result is being calculated. Every regulation which has conditions fulfilled and is not canceled is taken into account in the analysis result.

The food product analysis result is calculated as the sum of the obtained values divided by the sum of the maximum possible "success" values of all active regulations. The result is truncated to a range of minimum 0% to maximum 100% so that the result value can be easily understood.

Provided that the result of the regulation may have not only a numerical value but also a special value:
a) full food product acceptance (final analysis result would be 100%)
b) full food product rejection (final analysis result would be 0%)

This means that it may happen that only 1 regulation out of many activated regulations will be decisive for awarding the maximum score (100%) or the minimum score (0%) for a given food product.

The analysis result of product or multiple products is presented in the client application after the analysis is performed. The output file of the analysis towards diet configuration contains:
a) final score (numerical value from 0% to 100%)
b) diet configuration data along with intermediate results for each regulation and each tolerance

Diet event is a file defining dietary parameters for a given activation factor. The basic activation factor is time trigger, but it can be any phenomenon (for example: user enters into a specific geographic area or notification on the user device about the user's current financial status). Diet event is an abstract entity that defines the user's temporary dietary rules. Diet event has 2 types of sentences:
a) regulation - a set of sentences that generates the component result of the analysis.
b) functional - a set of sentences that generates the component result of the analysis, but also can be used as sample input data for user.

Diet event can be described by different type of event:
a) consumption recommendation (e.g. meal, breakfast, lunch)
b) consumption prohibition (e.g. all-day fasting)

For example, a short diet event (e.g. lasting 5 minutes) would typically determine features of a single moment of food consumption (e.g. one meal), whereas a long diet event (e.g. lasting 7 days) would typically determine features of many different moments of food consumption (e.g. every meal for 7 days).

The regulations in the diet event have the same structure as in the diet configuration. They may, but do not have to, have a set of conditions. They must have a set of rules. The set of conditions contains logical sentences connected by logical connectors. The set of rules contains logical sentences connected by logical connectors. Each regulation has defined intermediate results for conditions fulfillment and rules fulfillment.

In addition, diet event may contain functional sentences. A functional sentence comes down to applying the logic of "it is recommended to use" or "it is forbidden to use" for a specific entity. The entity may be a specific product or a specific food category. The entity may have defined value or value range. Examples of functional sentences are:
a) It is recommended to use 500 to 700 mL (default 600 mL) of milk
b) It is forbidden to use more than 100 mL of milk

Each functional statement has its intermediate result for states identical to those in the case of regulation:
a) success - fulfilled sentence
b) fail - not fulfilled sentence
c) undetermined - the sentence is impossible to determine

In addition to its analytical function, a diet event can act as an example or a suggestion for consumption. If the event has specific food products defined, the user can treat them as a recommendation to consume those during that event. This way user can immediately go to the food product analysis towards diet event using food products from this event.

The user can assign selected diet events to the account. At any time in the client application, the user can view the list of diet events and trigger the product analysis for the selected diet event. Additionally, the system can present a diet event to the user once an activation factor is met. For example, if the activation factor is geographic area X and the user's position is monitored by the client application, then when the user enters geographic area X, the client application can present the desired diet event.

The system can present diet events sorted by the nearest activation factor to user. If diet events are activated by time interval, the closest diet events will be those with a time interval closest to the user's selected time. If diet events are activated by geographic location, the closest diet events will be those with the geographic location closest to the user's selected geographic location.

Food product analysis towards diet event can be performed depending on the system operation mode: on the client device or on the server. The process of analyzing the diet event consists of 2 phases:
a) download necessary data for analysis
b) first iteration of data processing

The first phase is downloading necessary data for food product analysis. Regardless of where the analysis is processed (on the client or on the server), the necessary data must first be downloaded. The minimum set of data needed is:
a) diet configuration
b) food product description

Depending on your system settings and configuration settings, it may be possible or necessary to download additional data.

Next phase is to iteratively process the food product data towards diet event regulation and obtain the final result of the product analysis. Intermediate results are determined for each regulation and tolerance defined in the diet event. For each regulation defined in the diet event, intermediate results are calculated. For each regulation, a conditions fulfillment result and a rules fulfillment result are determined. In the case of an empty set of conditions, the entire regulation is always activated and calculations of the fulfillment of the rules are performed. Each functional sentence intermediate result is calculated and used in final analysis result.

The food product analysis result is calculated as the sum of the obtained values divided by the sum of the maximum possible "success" values of all active regulations and functional sentences. The result is truncated to a range of minimum 0% to maximum 100%.

The analysis result of product or multiple products is presented in the client application after the analysis is performed. The output file of the analysis towards diet event contains:
a) final score (numerical value from 0% to 100%)
b) diet event data along with intermediate results for each regulation and each functional sentence

### IMPLEMENTATION

The system can be used in many ways, both by an ordinary person in everyday life and by a specialist in their work:
1. A regular user can monitor and optimize their own food consumption - By using the system, the user consciously monitors the consumption of food products. The user acquires knowledge about the effect a given food product has on the body. With this knowledge, user can optimize consumption and respond appropriately to maintain the highest level of fitness and health (e.g. increasing physical activity after excessive energy consumption).
2. A regular user can monitor and optimize another person's food consumption (e.g. relative person with health problems or juvenile child) - Another important feature is the ability to analyze and monitor the consumption of food products by someone else than yourself. This is important in the case of people who cannot or do not know how to use modern technological solutions on their own. It can be for example unwell relative, young child or elderly person. By analyzing the consumption of food products, diet of this person can be optimized, ensuring better health.
3. A regular user can make conscious decisions about their consumption knowing the personalized rating of each food product - The system can help make decisions about what the user should or should not consume. Thanks to this, user can make better decisions in a store, restaurant or other place. This can result in a better financial situation for the user and help to minimize food waste.
4. A dietitian can monitor and optimize a patient's food consumption - The system can facilitate and improve the quality of a dietitian's work. The dietitian can program the diet configuration individually according to the needs of each patient. Then, dietitian can examine the patient's food consumptions and evaluate the dietary decisions they make.
5. A doctor in a medical facility (such as a hospital or clinic) can get to know patient's history of food consumption along with product ratings - An additional advantage is the ability of the medical unit to retroactively analyze the food products consumed by the patient who uses the system. In certain cases such as accident, illness or loss of consciousness, a person may not be able to explain their health condition and dietary needs. In such situations, the doctor is dependent on information from third parties, which may be absent or the information may be incorrect. Thanks to the use of the system by the user, the doctor would have reliable information immediately. In many situations, this means a greater chance of saving the patient's health or life.

## Claims

1. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet configuration is stored on the user device; and the food product analysis is performed on the user device using the stored diet configuration; and the food product analysis result is returned to the user device.

2. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet configuration is stored on the server; and the food product analysis is performed on the server using the stored diet configuration; and the food product analysis result is returned to the user device.

3. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet event is stored on the user device; and the food product analysis is performed on the user device using the stored diet event; and the food product analysis result is returned to the user device.

4. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet event is stored on the server; and the food product analysis is performed on the server using the stored diet event; and the food product analysis result is returned to the user device.

5. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet event is stored on the user device; and the diet event has an activation factor detectable on the user device; and the diet event is activated on the user device; and the activated diet event is returned to the user device.

6. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet event is stored on the server; and the diet event has an activation factor detectable on the server; and the diet event is activated on the server; and the activated diet event is returned to the user device.

7. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet event is stored on the server; and the diet event has an activation factor detectable on the user device; and the activation signal for diet event is detected on the user device; and the activation signal for diet event is send to the server; and the activated diet event is returned to the user device.

8. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet event is stored on the user device; and the diet event has an activation factor detectable by third party device or software; and the activation signal for diet event is detected; and the activation signal for diet event is send to user device; and the activated diet event is returned to the user device.

9. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the diet event is stored on the server; and the diet event has an activation factor detectable by third party device or software; and the activation signal for diet event is detected; and the activation signal for diet event is send to the server; and the activated diet event is returned to the user device.

10. The system of claim 2, wherein the food product analysis is using food product data received from server.

11. The system of claim 4, wherein the food product analysis is using food product data received from server.

12. The system of claim 2, wherein the food product analysis is using food product data received from third party.

13. The system of claim 4, wherein the food product analysis is using food product data received from third party.
